(19) Europäisches Patentamt
European Patent Office
Office européen des brevets

(11) EP 4 321 510 A1

(12) EUROPEAN PATENT APPLICATION
published in accordance with Art. 153(4) EPC

(43) Date of publication:
**14.02.2024 Bulletin 2024/07**

(21) Application number: **21936095.5**

(22) Date of filing: **30.11.2021**

(51) International Patent Classification (IPC):
***C07D 317/38*** *(2006.01)*

(52) Cooperative Patent Classification (CPC):
Y02E 60/10

(86) International application number:
**PCT/JP2021/043988**

(87) International publication number:
**WO 2022/215297 (13.10.2022 Gazette 2022/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.04.2021 JP 2021065963**

(71) Applicant: **Asahi Kasei Kabushiki Kaisha**
**Tokyo 1000006 (JP)**

(72) Inventor: **OCHI, Hiroyuki**
**Tokyo 100-0006 (JP)**

(74) Representative: **Strehl Schübel-Hopf & Partner**
**Maximilianstrasse 54**
**80538 München (DE)**

# (54) INDUSTRIAL PRODUCTION METHOD OF CYCLIC ALKYLENE CARBONATE

(57) The method for industrially producing a cyclic alkylene carbonate comprises: a first distillation and separation step of continuously introducing a crude cyclic alkylene carbonate into a low-boiling separation column A, and continuously withdrawing a column top component (At) through the column top and a column bottom component (Ab) containing a cyclic alkylene carbonate through the column bottom in the low-boiling separation column A; and a second distillation and separation step of continuously introducing the column bottom component (Ab) obtained in the first step into a cyclic alkylene carbonate purification column B having a side outlet, and continuously withdrawing three components of a column top component (Bt) through the column top, a side cut component (Bs) through the side outlet, and a column bottom component (Bb) through the column bottom in the purification column B, wherein the side cut component (Bs) is a cyclic alkylene carbonate with electronic grade specifications.

[Figure 1]

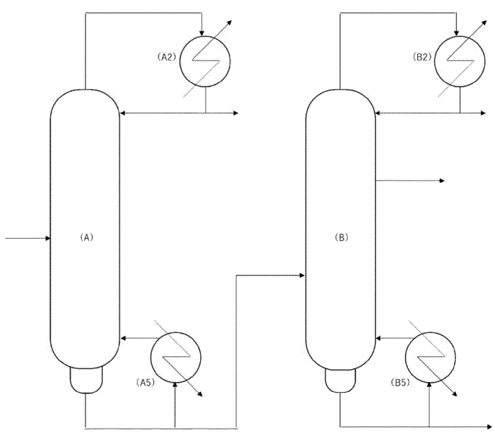




Processed by Luminess, 75001 PARIS (FR)

## Description

### Technical Field

The present invention relates to a method for industrially producing a cyclic alkylene carbonate.

### Background Art

Ethylene carbonate is also called 1,3-dioxolan-2-one and is excellent in various properties. For example, ethylene carbonate is an excellent polar organic solvent and an organic synthetic intermediate. Specifically, it is widely used, for example, in fields such as plastics, dyes, polymer synthesis, gas purification and separation, electronics industry, and organic synthesis industry. More specifically, it is mainly used as a spinning solution for acrylics, nylons, polyesters, polyvinyl chloride resins, or the like, for example, and is also used as a foaming agent for plastic and rubber intermediates, a soil stabilizer, a paint remover, an odor deodorant, a selective aromatic hydrocarbon extraction solvent, or a purification solvent for acidic gases in the synthetic ammonia industry, oil industry, or the like. Therefore, large-scale industrial production equipment for industrial production of ethylene carbonate has been developed. Ethylene carbonate is mainly used as a purification solvent for acidic gases such as ammonia source gas, city gas, and oil field gas; a liquid crystal solvent for electronics industry; a raw material for producing organic products such as furazolidone, vinylene carbonate, halogenated ethylene carbonate, dimethyl carbonate, and ethylene glycol.

In particular, high-purity ethylene carbonate is useful as a good solvent for lithium battery electrolyte solutions. In recent years, as the production of lithium batteries has greatly increased, the market demand for high-purity ethylene carbonate has also increased significantly. Accordingly, there is a need to develop high-purity ethylene carbonate that can be used as a good solvent for lithium battery electrolyte solutions.

Several methods for producing a high-purity cyclic alkylene carbonate have been proposed.

For example, Patent Document 1 proposes an ethylene carbonate purification process comprising the steps of delivering an ethylene carbonate crude product to a continuous rectification process, removing light-component impurities at the top, removing heavy-component impurities at the bottom, collecting an ethylene carbonate intermediate purified product at the side, buffering the ethylene carbonate intermediate purified product obtained in the aforementioned step, then delivering it to the batch rectification process, heating to establish the vapor-liquid equilibrium, then removing light-component materials at the top, and collecting an ethylene carbonate product at the side. The ethylene carbonate purification process of Patent Document 1 is thus a two-column system including continuous rectification and batch rectification, wherein the first column has a side outlet (side cut outlet) through which the ethylene carbonate intermediate purified product is collected, and the second column performs batch rectification. Also, Patent Document 2 proposes a rectification and control device for ethylene carbonate of electronic grade comprising a feed tank, an intermediate buffer tank, an electronic grade product can, a preliminary purification column and its attached equipment, and an electronic grade product column and its attached equipment, wherein a purification column liquid level meter and an intermediate buffer tank liquid level meter, and a product column liquid level meter set automatic control of the liquid levels, a purification column heating control valve group and a purification column reflux flow meter set chain self-control, and a product column heating control valve group and a product column reflux flow meter set chain self-control, for example. The rectification and control device for electronic grade ethylene carbonate of Patent Document 2 is a two-column system composed of two continuous rectifications, wherein the first column has a side outlet (side cut outlet), through which the ethylene carbonate intermediate purified product is collected.

### List of Prior Art Documents

#### Patent Document

Patent Document 1: Chinese patent application publication No. 106588862
Patent Document 2: Chinese utility model No. 208260225

### Summary of Invention

#### Problems to be Solved by Invention

However, in the ethylene carbonate purification process of Patent Document 1, since the second column performs batch rectification, the final ethylene carbonate yield is insufficient, and there is room for improvement. The

rectification and control device for electronic grade ethylene carbonate of Patent Document 2 has both columns that are of continuous type, but since the ethylene carbonate intermediate purified product is collected through the side outlet of the first column, the height of the first column needs to be high, thus the capital investment is large, and the energy efficiency of the entire purification column decreases, leaving room for improvement.

Then, in the conventional methods of Patent Documents 1 and 2, since the purity of crude ethylene carbonate in the liquid introduced into the first column is 99.7 mass% or more regardless of whether the purification column is of continuous type or batch type, other equipment is required to purify the crude ethylene carbonate to such an introduction purity, and there is room for improvement.

Therefore, an object of the present invention is to provide a production method that enables cyclic alkylene carbonate with electronic grade specifications to be produced efficiently on an industrial scale.

Means for Solving Problems

As a result of dedicated studies to solve the aforementioned problems, the inventor has found that cyclic alkylene carbonate with electronic grade specifications can be produced efficiently on an industrial scale by purifying crude cyclic alkylene carbonate in specific distillation and separation steps, thereby accomplishing the present invention.

That is, the present invention relates to the following aspects.

[1] A method for industrially producing a cyclic alkylene carbonate, comprising: a first distillation and separation step of continuously introducing a crude cyclic alkylene carbonate into a low-boiling separation column A, and continuously withdrawing a column top component (At) through a column top and a column bottom component (Ab) containing a cyclic alkylene carbonate through a column bottom in the low-boiling separation column A; and a second distillation and separation step of continuously introducing the column bottom component (Ab) obtained in the first distillation and separation step into a cyclic alkylene carbonate purification column B having a side outlet (side cut outlet), and continuously withdrawing three components of a column top component (Bt) through a column top, a side cut component (Bs) through the side outlet, and a column bottom component (Bb) through a column bottom in the purification column B, wherein the side cut component (Bs) is a cyclic alkylene carbonate with electronic grade specifications.

[2] The production method according to [1], wherein the crude cyclic alkylene carbonate is a crude ethylene carbonate.

[3] The production method according to [1] or [2], wherein the crude cyclic alkylene carbonate is a crude ethylene carbonate that is obtained using ethylene oxide and carbon dioxide as starting materials.

[4] The production method according to any one of [1] to [3], wherein, when a body column bottom liquid retention (L) of the low-boiling separation column A is defined as VA, a body column bottom liquid retention (L) of the purification column B is defined as VB, and an amount of the side cut component (Bs) to be withdrawn (t/hour) is defined as BsV, the conditions of formulas (1) and (2) below are satisfied:

$$10 \leq VA/BsV \leq 1000 \cdots (1);$$

and

$$10 \leq VB/BsV \leq 1000 \cdots (2).$$

[5] The production method according to any one of [1] to [4], wherein, when a column kettle inner diameter (cm) of the low-boiling separation column A is defined as Da, a column kettle inner diameter (cm) of the purification column B is defined as Db, and the amount of the side cut component (Bs) to be withdrawn (t/hour) is defined as BsV, the conditions of formulas (3) and (4) below are satisfied:

$$10 \leq Da/BsV \leq 50 \cdots (3);$$

and

$$10 \leq Db/BsV \leq 50 \cdots (4).$$

[6] The production method according to any one of [1] to [5], wherein, when a column body inner diameter (cm) of

the low-boiling separation column A is defined as DA, the column kettle inner diameter (cm) of the low-boiling separation column A is defined as Da, a column body inner diameter (cm) of the purification column B is defined as DB, and the column kettle inner diameter (cm) of the purification column B is defined as Db, the conditions of formulas (5) and (6) below are satisfied:

$$2 \leq DA/Da \leq 10 \cdots (5);$$

and

$$2 \leq DB/Db \leq 10 \cdots (6).$$

[7] The production method according to any one of [1] to [6], wherein a reboiler type in the low-boiling separation column A and/or the purification column B is of forced circulation, cross pipe falling film, or thin film evaporation.
[8] The production method according to any one of [1] to [7], wherein a regular packing is packed in the low-boiling separation column A and/or the purification column B, and the regular packing is any of Mellapak, Gempak, Technopack, Flexipac, Sulzer packing, Goodroll packing, Glitch grid, and gauze packing.

Advantages of Invention

According to the production method of the present invention, a cyclic alkylene carbonate with electronic grade specifications can be produced efficiently on an industrial scale.

Brief Description of Drawings

[Figure 1] Figure 1 is a schematic view of an example of the process flow of the method for industrially producing a cyclic alkylene carbonate of the present invention.
[Figure 2] Figure 2 is a schematic view of an example of the process flow of a conventional method for industrially producing a cyclic alkylene carbonate.

Mode for Carrying Out Invention

Hereinafter, an embodiment for carrying out the present invention (which will be hereinafter referred to as the "present embodiment") will be described in detail with reference to the figures, as required, but the present invention is not limited to this, and various modifications are possible without departing from the gist of the invention. In the figures, the positional relationships such as up, down, left, and right are based on the positional relationships shown in the figures unless otherwise specified. Furthermore, the dimensional ratios of the figures are not limited to the illustrated ratios.
The method for industrially producing a cyclic alkylene carbonate of the present embodiment comprises: a first distillation and separation step of continuously introducing a crude cyclic alkylene carbonate into a low-boiling separation column A, and continuously withdrawing a column top component (At) through the column top and a column bottom component (Ab) containing a cyclic alkylene carbonate through the column bottom in the low-boiling separation column A; and a second distillation and separation step of continuously introducing the column bottom component (Ab) obtained in the first distillation and separation step into a cyclic alkylene carbonate purification column B having a side outlet (side cut outlet) (which will be hereinafter referred to simply as "purification column B"), and continuously withdrawing three components of a column top component (Bt) through the column top, a side cut component (Bs) through the side outlet, and a column bottom component (Bb) through the column bottom in the purification column B, wherein the side cut component (Bs) is a cyclic alkylene carbonate with electronic grade specifications.
The method for industrially producing a cyclic alkylene carbonate of the present embodiment enables a cyclic alkylene carbonate with electronic grade specifications to be produced efficiently on an industrial scale by purifying a crude cyclic alkylene carbonate in specific distillation and separation steps. Specifically, a method of continuously introducing a crude ethylene carbonate into the low-boiling separation column A without withdrawal through the side cut and continuously withdrawing the column top component (At) through the column top and the column bottom component (Ab) containing a cyclic alkylene carbonate through the column bottom in the low-boiling separation column A allows the low-boiling separation column A (first column) to be specialized in removing the low-boiling component, and thus the column height can be shortened, so that capital investment can be reduced. Then, both the low-boiling and high-boiling components that remain in slight amounts can be removed by continuously introducing the column bottom component (Ab) into the purification column B having the side outlet and continuously withdrawing three components of the low-boiling component (column top component (Bt)) through the column top, ethylene carbonate (side cut component (Bs)) through the side outlet, and the high-boiling component (column bottom component (Bb)) through the column bottom in the purification column B, so that a cyclic alkylene carbonate of electronic grade can be produced. Then, since most of the low-boiling component has been removed in the low-boiling separation column A (first column), there is room in the column load in the purification column B (second column), and the energy consumption for separation can be decreased. Further, since the pressure loss in the column can be reduced due to no increase in column load, and the temperature at the column bottom can be lowered, the amounts of the low-boiling and high-boiling by-products generated by decomposition, polymerization reaction, and the like of the cyclic alkylene carbonate can be reduced in the purification column B, as a result of which the amount of waste can be suppressed.

In the present embodiment, a cyclic alkylene carbonate with electronic grade specifications refers to a cyclic alkylene carbonate with a purity of 99.97 mass% or more, in which the content of each of the three components of alkylene oxide, alkylene glycol, and dialkylene glycol is 50 mass ppm or less, the water content is 50 mass ppm or less, and each of the nine metal components of sodium, potassium, copper, iron, lead, zinc, chromium, nickel, and calcium is 1 μg/mL or less.

Further, in the present embodiment, the phrase "an industrial scale" refers to a scale in which a cyclic alkylene carbonate is produced at a ratio of 1 t (ton)/hour or more, preferably 2 t (tons)/hour or more, more preferably 3 t (tons)/hour or more, further preferably 4 t (tons)/hour or more. The upper limit of the industrial scale is not specifically limited but is, for example, 15 t (tons)/hour or less.

The crude cyclic alkylene carbonate to be continuously introduced into the low-boiling separation column A is preferably a crude ethylene carbonate.

Further, the crude cyclic alkylene carbonate to be continuously introduced into the low-boiling separation column A is preferably a crude ethylene carbonate to be obtained by using ethylene oxide and carbon dioxide as starting materials.

Further, the purity of the crude cyclic alkylene carbonate to be continuously introduced into the low-boiling separation column A may be 99.5 mass% or less. Conventionally, the purity of the crude cyclic alkylene carbonate to be continuously introduced into the low-boiling separation column A needs to be 99.7 mass% or more in order to obtain a cyclic alkylene carbonate with electronic grade specifications. In the production method of the present embodiment, even if the purity of the crude cyclic alkylene carbonate to be continuously introduced into the low-boiling separation column A is 99.5 mass% or less, the low-boiling separation column A is specialized in removing the low-boiling component, thereby enabling sufficient distillation and separation, so that a cyclic alkylene carbonate with electronic grade specifications can be eventually obtained.

The crude cyclic alkylene carbonate to be continuously introduced into the low-boiling separation column A may be a crude ethylene carbonate obtained by the Asahi Kasei method, that is, a crude ethylene carbonate with a purity of 99.5 mass% that is obtained using ethylene oxide (EO) and carbon dioxide ($CO_2$) as starting materials. Since the low-boiling separation column A is specialized in removing the low-boiling component, there is room in the column load, and thus even if the introduced liquid into the low-boiling separation column A is a crude ethylene carbonate with specifications equivalent to an industrial grade with a purity of ethylene carbonate of 99.5 mass%, sufficient distillation and separation are possible.

The Asahi Kasei method refers to the method for producing a cyclic alkylene carbonate according to WO 2004/108696. Specifically, it is a method for producing a cyclic alkylene carbonate by reacting alkylene oxide with carbon dioxide in the presence of a catalyst in a reactor, wherein the reactor communicates with a process-side flow path of a heat exchanger via piping to form a circulation circuit, the heat exchanger has a heat exchange-side flow path for allowing a heat exchange medium with a temperature adjusted to a predetermined temperature range to flow and the process-side flow path for allowing a process liquid that is heat-exchanged in relation to the production of alkylene carbonate to flow, and the production method maintains the internal temperature of the process-side flow path at 135 to 200°C by allowing the process liquid to flow through the circulation circuit including the reactor and the process-side flow path of the heat exchanger, while allowing a heat exchange medium with a temperature of 140 to 200°C to flow through the heat exchange-side flow path of the heat exchanger during the reaction or after the reaction.

Hereinafter, an example of the method for producing a crude cyclic alkylene carbonate to be used in the present embodiment will be described.

The alkylene oxide to be used in the method for producing a crude cyclic alkylene carbonate as a starting material is, for example, a compound represented by formula (1) below:

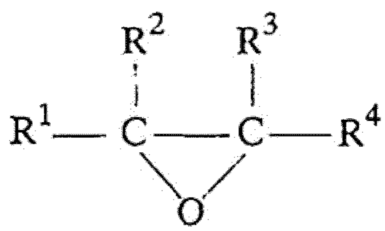

(1)

wherein $R^1$, $R^2$, $R^3$, and $R^4$ are each independently a hydrogen atom, a linear hydrocarbon group having 1 to 8 carbon atoms, an alicyclic hydrocarbon group having 3 to 8 carbon atoms, or an aromatic hydrocarbon group having 6 to 8 carbon atoms.

Examples of the alkylene oxide include ethylene oxide, propylene oxide, butylene oxide, vinyl ethylene oxide, cyclohexene oxide, and styrene oxide. In particular, ethylene oxide and propylene oxide are preferable in view of ease of availability.

In the production method for crude cyclic alkylene carbonate, it is preferable to react the alkylene oxide with carbon dioxide in the presence of a catalyst in the reactor, to obtain a reaction mixture in the reactor. The reaction mixture contains a cyclic alkylene carbonate represented by formula (2) below:

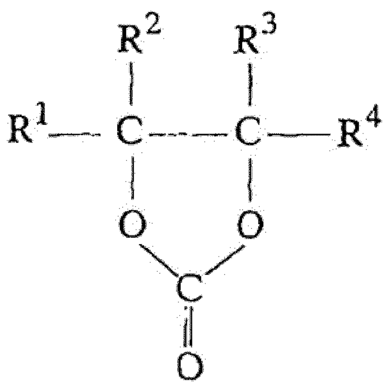

(2)

wherein $R^1$, $R^2$, $R^3$, and $R^4$ are as defined in formula (1) .

In the present embodiment, specific examples of the cyclic alkylene carbonate are not specifically limited, but examples thereof include ethylene carbonate, propylene carbonate, butylene carbonate, vinyl ethylene carbonate, cyclohexene carbonate, and styrene carbonate, and ethylene carbonate and propylene carbonate are preferable.

In the production method for crude cyclic alkylene carbonate, the reaction to obtain the cyclic alkylene carbonate from alkylene oxide and carbon dioxide is represented by formula (3) below:

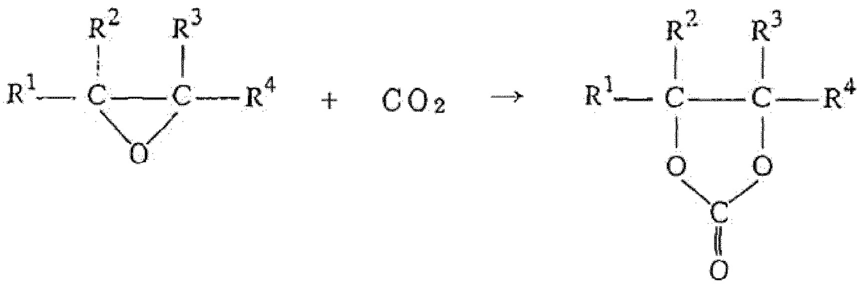

(3)

wherein $R^1$, $R^2$, $R^3$, and $R^4$ are as defined in formula (1) .

In the production method for crude cyclic alkylene carbonate, the catalyst to be used in the reaction between alkylene oxide and carbon dioxide is not specifically limited, as long as it is a catalyst commonly used for implementing the reaction of formula (3) above. Specifically, halides such as tetraethylammonium bromide and 5-membered/6-membered ring hydrocarbon, organic catalysts such as ammonium rhodanide or its pyrolysis product, inorganic catalysts such as bromides and iodides of metals or alkali metals, and a small amount of alcohols or water added to these are used, and inorganic catalysts that are easy to recover catalyst are preferable. The amount of the catalyst to be used is

not specifically limited but is preferably 0.1 to 3 mass%, further preferably 0.1 to 2 mass%, based on the reaction system.

In the production method for crude cyclic alkylene carbonate, the temperature in the reaction between alkylene oxide and carbon dioxide is preferably 100 to 200°C, more preferably 150 to 190°C. The reaction pressure is preferably 2 to 15 MPa, more preferably 4 to 12 MPa. The reaction time differs depending on the composition ratio of alkylene oxide and carbon dioxide that are starting materials, the type of alkylene oxide, the type and concentration of the catalyst used, the reaction temperature and the like. For example, when the average retention time determined from the retained liquid volume and the total liquid supply volume in a complete mixing reactor is defined as the reaction time, it is generally 0.5 to 10 hours, preferably 1 to 5 hours.

In implementing the production method for crude cyclic alkylene carbonate, the quantitative ratio of alkylene oxide and carbon dioxide that are starting materials, in terms of a molar ratio of carbon dioxide to alkylene oxide, is generally 1 to 5, preferably 1 to 2. In general, when excess carbon dioxide gas is released from the reactor, accompanying unreacted alkylene oxide also increases. Accordingly, when adjusting the quantitative ratio of alkylene oxide and carbon dioxide, a method of adjusting the amount of carbon dioxide to be fed so that the reactor pressure is constant is preferable, instead of releasing excess carbon dioxide gas from the reactor.

The reactor for implementing the reaction in the method for producing a crude cyclic alkylene carbonate preferably communicates with the process-side flow path of the heat exchanger via piping to form a circulation circuit.

As the reaction system in the production method for crude cyclic alkylene carbonate, commonly used reaction systems such as a complete mixing reactor, multistage reaction system using complete mixing reactors connected in series, a plug flow reactor, and a method combining a complete mixing reactor and a plug flow reactor can be used.

In the case of producing crude cyclic alkylene carbonate using a complete mixing reactor, a method of circulating a reaction solution with a large flow rate using a pump is preferable so that carbon dioxide is easily dissolved in the reaction mixture. In general, the number of cycles per unit time is 10 to 50 cycles/hour, preferably 20 to 40 cycles/hour. In the case of removing the heat of reaction by providing a heat exchanger in the course of piping for pumping and circulating the reaction mixture, circulation with a large flow rate is preferable since the cooling capacity of the heat exchanger increases.

The heat exchanger to be used for the method for producing a crude cyclic alkylene carbonate preferably has a heat exchange-side flow path for allowing a heat exchange medium with a temperature adjusted to a predetermined temperature range to flow and a process-side flow path for allowing a process liquid to be heat-exchanged in relation to the production of crude cyclic alkylene carbonate to flow. The process liquid is a liquid processed by a heat exchanger (that is, with a temperature adjusted), and the heat exchange medium is a medium for adjusting the temperature of the process liquid. Then, the heat exchange-side flow path of the heat exchanger is a flow path for allowing the heat exchange medium to flow, and the process-side flow path is a flow path for allowing the process liquid to flow.

The heat exchanger to be used in the method for producing a crude cyclic alkylene carbonate is preferably capable of maintaining the internal temperature of the process-side flow path at 135°C to 200°C by allowing a heat exchange medium at a temperature of 140°C to 200°C to flow through the heat exchange-side flow path of the heat exchanger. For example, a corrugated-tube heat exchanger, a double-tube heat exchanger, a common multitubular heat exchanger, and the like to be provided inside the reactor can be used individually or in combination. As the heat exchanger, a multitubular heat exchanger that is capable of increasing the heat transfer area and reducing the size of the device is preferably used.

In the case of using a multitubular heat exchanger, any of the tube side and the shell side of the multitubular heat exchanger may be used as the heat exchange-side flow path or process-side flow path of the process liquid and the heat exchange medium. What kind of liquid is passed through each of the tube side and the shell side of the heat exchanger may be appropriately selected, as required, depending on the case such as the case of increasing the overall heat transfer coefficient (U) to use a small heat exchanger and the case of facilitating cleaning by passing a fluid to which contaminants easily adhere through the tube side.

The heat exchanger used in the method for producing a crude cyclic alkylene carbonate is preferably a device that functions as both a preheater and a cooler. Such a heat exchanger can be used as a preheater for raising the temperature of the reaction solution to the reaction initiation temperature at the startup and can be used as a cooler for removing the reaction heat during steady operation.

The material of the process-side flow path of the heat exchanger is not specifically limited, as long as it has a corrosion resistance to the process liquid. It is preferable to use stainless steel because iron rust causes formation of an alkylene oxide polymer by its catalytic action.

The heat exchange medium to be used in the method for producing a crude cyclic alkylene carbonate is preferably one capable of maintaining its temperature at 140°C to 200°C, preferably 140°C to 180°C. Examples thereof include water, steam, and heat medium oil, which are generally used as heat exchange media. As the heat exchange medium, heat medium oil, which is thermally stable and has a low vapor pressure, is preferable because the design pressure of the heat exchanger can be lowered. Furthermore, since the temperature of heat medium oil is easy to adjust, the reaction temperature can be adjusted by increasing or decreasing the amount of the heat exchange medium to be

fed when it is used as a heat exchange medium, thereby facilitating the operation of the production device of the crude cyclic alkylene carbonate.

Figure 1 is a schematic view of an example of the process flow of the method for industrially producing a cyclic alkylene carbonate of the present embodiment. In Figure 1, A is a low-boiling separation column that continuously distills and separates cyclic alkylene carbonate from the low-boiling component, and B is a purification column that continuously distills and separates the cyclic alkylene carbonate into three components of a column top component (Bt), a side cut component (Bs), and a column bottom component (Bb). Further, A2 is a column top condenser of the low-boiling separation column A, and A5 is a reboiler of the low-boiling separation column A. Likewise, B2 is a column top condenser of the purification column B, and B5 is a reboiler of the purification column B.

In the method for industrially producing a cyclic alkylene carbonate of the present embodiment, since the component containing a cyclic alkylene carbonate is thus withdrawn through the column bottom in the low-boiling separation column A instead of through the side outlet, the column height can be shortened, and the capital investment can be reduced. Further, since the low-boiling separation column A is specialized in removing the low-boiling component, there is room in the column load, and the energy cost can also be reduced. The yield of the cyclic alkylene carbonate finally obtained is also improved.

In the production method of the present embodiment, the first distillation and separation step is a step of continuously introducing a crude cyclic alkylene carbonate into a low-boiling separation column A, and continuously withdrawing a column top component (At) through the column top and a column bottom component (Ab) containing a cyclic alkylene carbonate through the column bottom in the low-boiling separation column A.

The material of the low-boiling separation column A is not specifically limited, but examples thereof include carbon steel, stainless steel, and high alloy steel. In particular, stainless steel is preferable.

In the low-boiling separation column A, the theoretical number of stages is preferably 10 to 50 stages, more preferably 15 to 40 stages, further preferably 20 to 30 stages. In the low-boiling separation column A, the crude cyclic alkylene carbonate is preferably introduced from the 1st to 40th stage positions, more preferably from the 3rd to 30th stage positions, further preferably from the 5th to 20th stage positions, from the top of the theoretical stages.

In the low-boiling separation column A, the amount of the crude cyclic alkylene carbonate to be introduced is preferably 1500 to 15000 kg/hour, more preferably 3000 to 14000 kg/hour, further preferably 4000 to 13500 kg/hour.

In the low-boiling separation column A, the low-boiling component (column top component (At)) are withdrawn through the column top. The flow rate of the column top component (At) to be withdrawn is preferably 135 to 1500 kg/hour, more preferably 250 to 1450 kg/hour, further preferably 300 to 1400 kg/hour.

In the low-boiling separation column A, the column bottom component (Ab) containing cyclic alkylene carbonate is withdrawn through the column bottom. The flow rate of the column bottom component (Ab) to be withdrawn is preferably 1300 to 14500 kg/hour, more preferably 3000 to 14000 kg/hour, further preferably 4000 to 13500 kg/hour.

In the production method of the present embodiment, since the low-boiling separation column A is specialized in removing the low-boiling component, the low-boiling component can be sufficiently separated from the introduced liquid even with specifications equivalent to an industrial grade with a purity of the cyclic alkylene carbonate, and cyclic alkylene carbonate containing less low-boiling component can be obtained in the column bottom component (Ab).

For the operating conditions of the low-boiling separation column A, the reflux ratio at the column top is preferably 1 to 40, more preferably 5 to 30, further preferably 10 to 20. In the low-boiling separation column A, the reflux volume at the column top is preferably 200 to 60000 kg/hour, more preferably 1250 to 43500 kg/hour, further preferably 3000 to 28000 kg/hour. In the low-boiling separation column A, the temperature at the column bottom is preferably 100 to 150°C, more preferably 110 to 140°C, further preferably 120 to 130°C. In the production method of the present embodiment, since the component containing cyclic alkylene carbonate is withdrawn through the column bottom instead of through the side outlet in the low-boiling separation column A, so that the pressure loss in the column can be reduced due to no increase in column load, and the temperature at the column bottom can be lowered, the amounts of the low-boiling and high-boiling by-products generated by decomposition, polymerization reaction, and the like of the cyclic alkylene carbonate can be reduced, as a result of which the amount of waste can be suppressed. In the low-boiling separation column A, the amount of steam to be used in the reboiler is preferably 100 to 17600 kg/hour, more preferably 350 to 13000 kg/hour, further preferably 800 to 9000 kg/hour. In the low-boiling separation column A, the component containing cyclic alkylene carbonate is withdrawn through the column bottom instead of through the side outlet, so that the pressure loss in the column can be reduced due to no increase in column load, the temperature at the column bottom can be lowered, the amount of steam to be used can be reduced.

In the production method of the present embodiment, the second distillation and separation step is a step of continuously introducing the column bottom component (Ab) obtained in the first distillation and separation step into the purification column B having the side outlet and continuously withdrawing three components of a column top component (Bt) through the column top, a side cut component (Bs) through the side outlet, and a column bottom component (Bb) through the column bottom in the purification column B. Then, a cyclic alkylene carbonate with electronic grade specifications can be obtained through the side outlet of the purification column B.

The material of the purification column B is not specifically limited, but examples thereof include carbon steel, stainless steel, and high alloy steel. In particular, stainless steel is preferable. In the purification column B, the theoretical number of stages is preferably 5 to 30 stages, more preferably 6 to 20 stages, further preferably 7 to 15 stages. In the purification column B, the column bottom component (Ab) of the low-boiling separation column A is preferably introduced from the 1st to 25th stage positions, more preferably the 2nd to 15th stage positions, further preferably the 3 to 10 stage positions, from the top of the theoretical stages.

In the purification column B, the side outlet is preferably located at the 1st to 25th stage positions, more preferably the 2nd to 15th stage positions, further preferably the 3rd to 10th stage positions, from the top of the theoretical stages.

For the operating conditions of the purification column B, the reflux ratio at the column top is preferably 10 to 100, more preferably 50 to 90, further preferably 60 to 80. In the purification column B, the reflux volume at the column top is preferably 400 to 25000 kg/hour, more preferably 2500 to 21000 kg/hour, further preferably 3600 to 16000 kg/hour. In the purification column B, the low-boiling component (column top component (Bt)) is withdrawn through the column top. The flow rate of the column top component (Bt) to be withdrawn is preferably 25 to 250 kg/hour, more preferably 50 to 230 kg/hour, further preferably 60 to 200 kg/hour. In the purification column B, the high-boiling component (column bottom component (Bb)) is withdrawn through the column bottom. The flow rate of the column bottom component (Bb) to be withdrawn is preferably 15 to 150 kg/hour, more preferably 25 to 140 kg/hour, further preferably 30 to 130 kg/hour. In the purification column B, a cyclic alkylene carbonate is withdrawn through the side outlet as the side cut component (Bs). The flow rate of the side cut component (Bs) to be withdrawn is preferably 1300 to 14000 kg/hour, more preferably 2500 to 13000 kg/hour, further preferably 3000 to 12500 kg/hour.

The cyclic alkylene carbonate to be obtained as the side cut component (Bs) is a cyclic alkylene carbonate with electronic grade specifications with a purity of 99.97 mass% or more, in which the content of each of the three components of alkylene oxide, alkylene glycol, and dialkylene glycol is 50 mass ppm or less, the water content is 50 mass ppm or less, and each of the nine metal components of sodium, potassium, copper, iron, lead, zinc, chromium, nickel, and calcium is 1 μg/mL or less, as described above.

In the present embodiment, the purity of the cyclic alkylene carbonate and the content of the metal components can be measured by the methods according to Examples, which will be described below.

In the purification column B, the temperature at the column bottom is preferably 100 to 150°C, more preferably 110 to 140°C, further preferably 120 to 130°C. In the production method of the present embodiment, since the low-boiling component is sufficiently removed in the low-boiling separation column A, so that the column load can be reduced, and the temperature at the column bottom can be lowered in the purification column B, the amounts of the low-boiling and high-boiling by-products generated by decomposition, polymerization reaction, and the like of the cyclic alkylene carbonate can be reduced, as a result of which the amount of waste can be suppressed. In the purification column B, the amount of steam to be used in the reboiler is preferably 100 to 7300 kg/hour, more preferably 600 to 6100 kg/hour, further preferably 900 to 4700 kg/hour. In the production method of the present embodiment, since the low-boiling component is sufficiently removed in the low-boiling separation column A, so that the column load can be reduced, and the temperature at the column bottom can be lowered, in the purification column B, the amount of steam to be used can be reduced.

As described above, a cyclic alkylene carbonate of electronic grade can be produced in the second distillation and separation step by continuously introducing the column bottom component (Ab) into the purification column B and withdrawing the cyclic alkylene carbonate through the side outlet so that slight amounts of the low-boiling and high-boiling components remaining can be removed. Then, since most of the low-boiling component has been removed in the low-boiling separation column A (first column), there is room in the column load in the purification column B (second column), and the energy consumption for separation can be decreased. Further, since the pressure loss in the column can be reduced due to no increase in column load, and the temperature at the column bottom can be lowered, the amounts of the low-boiling and high-boiling by-products generated by decomposition, polymerization reaction, and the like of the cyclic alkylene carbonate can be reduced in the purification column B, as a result of which the amount of waste can be suppressed.

In the production method of the present embodiment, a cyclic alkylene carbonate with electronic grade specifications can be produced efficiently and stably over a long period of time on an industrial scale.

In the steady state of the continuous operation of the method for producing a cyclic alkylene carbonate of the present embodiment, when the body column bottom liquid retention (L) of the low-boiling separation column A is defined as VA, the body column bottom liquid retention (L) of the purification column B is defined as VB, and the amount of the side cut component (Bs) to be withdrawn (t/hour) is defined as BsV, the conditions of formulas (1) and (2) below are preferably satisfied.

$$10 \leq VA/BsV \leq 1000 \cdots (1)$$

$$10 \leq VB/BsV \leq 1000 \cdots (2)$$

For example, in the case of using a production device at BsV = 1.325 t/hour, the device is normally operated with VA and VB set to about 1,500 L, so as to fall within a range of about VA/BsV = 1132 and VB/BsV = 1132 for the ease of control of the low-boiling separation column A and the purification column B. When the conditions of formula (1) and (2) above are satisfied, the amounts of the low-boiling and high-boiling by-products generated by decomposition, polymerization reaction, and the like of the cyclic alkylene carbonate tends to be suppressed. From the same point of view, VA/BsV is more preferably 20 to 500, further preferably 30 to 200, and VB/BsV is more preferably 20 to 500, further preferably 30 to 200.

In the present embodiment, the steady state of the continuous operation means the state where each flow rate is stable in the predetermined ranges. In general, a steady state is reached 72 hours or more after the starting materials are added.

In the steady state of the continuous operation of the method for producing a cyclic alkylene carbonate of the present embodiment, when the column kettle inner diameter (cm) of the low-boiling separation column A is defined as Da, the column kettle inner diameter (cm) of the purification column B is defined as Db, and the amount of the side cut component (Bs) to be withdrawn (t/hour) is defined as BsV, the conditions of formulas (3) and (4) below are preferably satisfied.

$$10 \leq Da/BsV \leq 50 \cdots (3)$$

$$10 \leq Db/BsV \leq 50 \cdots (4)$$

When the conditions of formula (3) and (4) above are satisfied, the amounts of the low-boiling and high-boiling by-products generated by decomposition, polymerization reaction, and the like of the cyclic alkylene carbonate tends to be suppressed. From the same point of view, Da/BsV is more preferably 12 to 40, further preferably 15 to 30, and Db/BsV is more preferably 12 to 40, further preferably 15 to 30.

In the method for producing a cyclic alkylene carbonate of the present embodiment, when the column body inner diameter (cm) of the low-boiling separation column A is defined as DA, the column kettle inner diameter (cm) of the low-boiling separation column A is defined as Da, the column body inner diameter (cm) of the purification column B is defined as DB, and the column kettle inner diameter (cm) of the purification column B is defined as Db, the conditions of formulas (5) and (6) below are preferably satisfied.

$$2 \leq DA/Da \leq 10 \cdots (5)$$

$$2 \leq DB/Db \leq 10 \cdots (6)$$

When the conditions of formulas (5) and (6) above are satisfied, the amounts of the low-boiling and high-boiling by-products generated by decomposition, polymerization reaction, and the like of the cyclic alkylene carbonate tends to be suppressed. From the same point of view, DA/Da is more preferably 3 to 8, further preferably 3 to 7, and DB/Db is more preferably 3 to 8, further preferably 3 to 7.

In the present embodiment, the column body inner diameter of the low-boiling separation column A refers to the inner diameter (cm) at the position of the largediameter section over the entire length of the column, and the column kettle inner diameter of the low-boiling separation column A refers to the inner diameter (cm) at the position of the small-diameter section at the bottom of the column where the liquid is retained.

In the present embodiment, the reboiler type in the low-boiling separation column A and/or the purification column B is preferably of forced circulation, cross pipe falling film, or thin film evaporation. Use of such a reboiler can improve the heat exchange efficiency and reduce the temperature on the tube wall surfaces in contact with the process fluid, so that impurities generated due to decomposition and polymerization can be suppressed.

In the present embodiment, a regular packing is preferably packed in the low-boiling separation column A and/or the purification column B. Examples of the regular packing include those made of metals and resins. Mellapak, Gempak, Technopack, Flexipac, Sulzer packing, Goodroll packing, Glitch grid, or gauze packing is preferable.

Examples

Hereinafter, the present invention will be described more specifically by way of Examples and Comparative Examples. The present invention is not limited to the following Examples at all.

[Purity of ethylene carbonate]

The purity of the ethylene carbonate obtained in each of Examples and Comparative Examples was measured by gas chromatography disclosed in HG/T 5391.

[Content of each of alkylene oxide, alkylene glycol, and dialkylene glycol]

The content of each of the three components of alkylene oxide, alkylene glycol, and dialkylene glycol in the ethylene carbonate obtained in each of Examples and Comparative Examples was measured by gas chromatography disclosed in HG/T 5391.

[Water content]

The water content of the ethylene carbonate obtained in each of Examples and Comparative Examples was measured by the coulometric titration method disclosed in HG/T 5391.

[Content of each metal component]

The content of each of the nine metal components of sodium, potassium, copper, iron, lead, zinc, chromium, nickel, and calcium in the ethylene carbonate obtained in each of Examples and Comparative Examples was measured by the inductively coupled plasma (ICP) method disclosed in HG/T 5391.

[Example 1]

Figure 1 is a schematic view of the process flow of the production method for ethylene carbonate used in Example 1. In Figure 1, A is a low-boiling separation column that continuously distills and separates ethylene carbonate from the low-boiling component, and B is a purification column that continuously distills and separates the ethylene carbonate into three components of a column top component (Bt), a side cut component (Bs), and a column bottom component (Bb). A2 is a column top condenser of the low-boiling separation column A, and A5 is a reboiler of the low-boiling separation column A. Likewise, B2 is a column top condenser of the purification column B, and B5 is a reboiler of the purification column B. The reboiler type of the low-boiling separation column A was of forced circulation, and the reboiler type of the purification column B was of forced circulation. Further, the packings of the low-boiling separation column A and the purification column B were each gauze packing.

The introduced liquid continuously introduced into the low-boiling separation column A was a crude ethylene carbonate with specifications equivalent to an industrial grade with a purity of ethylene carbonate of 99.5 mass%. In Example 1, the material of the low-boiling separation column A was stainless steel, and the theoretical number of stages was 25 stages. The crude ethylene carbonate was introduced into the low-boiling separation column A from the 14th stage position from the top of the theoretical stages. The material of the purification column B was stainless steel, the theoretical number of stages was 9 stages, and it had a side outlet. In the purification column B, the column bottom component (Ab) of the low-boiling separation column A was introduced from the 7th stage position from the top of the theoretical stages. In the purification column B, the side outlet was located at the 3rd stage position from the top of the theoretical stages.

The crude ethylene carbonate was introduced into the low-boiling separation column A at a flow rate of 1500 kg/hour, and the flow rate of the low-boiling component (column top component (At)) to be withdrawn through the column top of the low-boiling separation column A was 135 kg/hour. The column bottom component (Ab) containing an ethylene carbonate with a purity of 99.73 mass% was withdrawn at a ratio of 1365 kg/hour through the column bottom of the low-boiling separation column A and introduced into the purification column B. In the purification column B, the flow rate of the low-boiling component (column top component (Bt)) to be withdrawn through the column top was 25 kg/hour, and the flow rate of the high-boiling component (column bottom component (Bb)) to be withdrawn through the column bottom was 15 kg/hour. In the purification column B, ethylene carbonate was withdrawn at a ratio of 1325 kg/hour (BsV = 1.325 t/hour) through the side outlet as the side cut component (Bs). In the purification column B, the ethylene carbonate withdrawn through the side outlet had a purity of 99.994 mass%, a diethylene glycol (hereinafter also referred to as "DEG") concentration of 15 mass ppm, a content of each of ethylene oxide and ethylene glycol of 30 mass ppm or less,

a water content of 20 mass ppm or less, and a content of each of the nine metal components of sodium, potassium, copper, iron, lead, zinc, chromium, nickel, and calcium of 1 μg/mL or less, thereby satisfying the specifications (electronic grade specifications) for electrolyte solution applications.

For the operating conditions of the low-boiling separation column A, the reflux ratio was 14.8, the reflux volume at the column top was 2000 kg/hour, and the temperature at the column bottom was 124°C. The amount of steam to be used in the reboiler of the low-boiling separation column A was 900 kg/hour.

For the operating conditions of the purification column B, the reflux ratio was 70.0, the reflux volume at the column top was 1750 kg/hour, and the temperature at the column bottom was 124°C. The amount of steam to be used in the reboiler of the purification column B was 670 kg/hour.

Further, in the steady state of the continuous operation of the ethylene carbonate purification of Example 1, when the body column bottom liquid retention (L) of the low-boiling separation column A is defined as VA, the body column bottom liquid retention (L) of the purification column B is defined as VB, the amount of the side cut component (Bs) to be withdrawn (t/hour) is defined as BsV, the column body inner diameter (cm) of the low-boiling separation column A is defined as DA, the column kettle inner diameter (cm) of the low-boiling separation column A is defined as Da, the column body inner diameter (cm) of the purification column B is defined as DB, and the column kettle inner diameter (cm) of the purification column B is defined as Db, VA/BsV was 73, VB/BsV was 73, Da/BsV was 19, Db/BsV was 19, DA/Da was 4.4, and DB/Db was 4.

In Example 1, the low-boiling separation column A (first column) was specialized in removing the low-boiling component by the method of continuously introducing crude ethylene carbonate into the low-boiling separation column A and continuously withdrawing the low-boiling component (column top component (At)) and the column bottom component (Ab) containing an ethylene carbonate for distillation and separation without withdrawal through the side cut, so that the column height could be shortened, and the capital investment could be reduced. Further, the low-boiling separation column A was specialized in removing the low-boiling component, so that there was room in the column load, and the introduced liquid could be sufficiently separated as crude ethylene carbonate with specifications equivalent to an industrial grade with a purity of ethylene carbonate of 99.5 mass%. Then, slight amounts of the low-boiling and high-boiling components remaining could be removed by continuously introducing the column bottom component (Ab) into the purification column B having the side outlet and withdrawing ethylene carbonate through the side outlet, so that ethylene carbonate of electronic grade could be produced. Then, since most of the low-boiling component has been removed in the low-boiling separation column A (first column), there was room for the column load in the purification column B (second column), and the energy consumption for separation could be decreased. Further, since the pressure loss in the column could be reduced due to no increase in column load, and the temperature at the column bottom could be lowered, the amounts of the low-boiling and high-boiling by-products generated by decomposition, polymerization reaction, and the like can be reduced in the purification column B, as a result of which the amount of waste could be reduced.

Thus, an ethylene carbonate with electronic grade specifications could be produced efficiently on an industrial scale in Example 1.

[Example 2]

In the same manner as in Example 1, ethylene carbonate was purified using the low-boiling separation column A and the purification column B according to the process flow shown in Figure 1. However, the packings of the low-boiling separation column A and the purification column B were each Technopack.

The introduced liquid continuously introduced into the low-boiling separation column A was a crude ethylene carbonate with specifications equivalent to an industrial grade with a purity of ethylene carbonate of 99.5 mass%. In Example 2, the material of the low-boiling separation column A was stainless steel, and the theoretical number of stages was 25 stages. In the low-boiling separation column A, the crude ethylene carbonate was introduced from the 14th stage position from the top of the theoretical stages. The material of the purification column B was stainless steel, the theoretical number of stages was 9 stages, and it had a side outlet. In the purification column B, the column bottom component (Ab) of the low-boiling separation column A was introduced from the 7th stage position from the top of the theoretical stages. In the purification column B, the side outlet was located at the 3rd stage position from the top of the theoretical stages.

The crude ethylene carbonate was introduced into the low-boiling separation column A at a flow rate of 5350 kg/hour, and the flow rate of the low-boiling component (column top component (At)) to be withdrawn through the column top of the low-boiling separation column A was 495 kg/hour. The column bottom component (Ab) containing an ethylene carbonate with a purity of 99.73 mass% was withdrawn at a ratio of 4855 kg/hour through the column bottom of the low-boiling separation column A and introduced into the purification column B. In the purification column B, the flow rate of the low-boiling component (column top component (Bt)) to be withdrawn through the column top was 95 kg/hour, and the flow rate of the high-boiling component (column bottom component (Bb)) to be withdrawn through the column bottom

was 45 kg/hour. In the purification column B, ethylene carbonate was withdrawn at a ratio of 4715 kg/hour (BsV = 4.715 t/hour) through the side outlet as the side cut component (Bs). In the purification column B, the ethylene carbonate withdrawn through the side outlet had a purity of 99.994 mass%, a DEG concentration of 18 mass ppm, a content of each of ethylene oxide and ethylene glycol of 30 mass ppm or less, a water content of 20 mass ppm or less, and a content of each of the nine metal components of sodium, potassium, copper, iron, lead, zinc, chromium, nickel, and calcium of 1 μg/mL or less, thereby satisfying the specifications (electronic grade specifications) for electrolyte solution applications.

For the operating conditions of the low-boiling separation column A, the reflux ratio was 14.9, the reflux volume at the column top was 7400 kg/hour, and the temperature at the column bottom was 124°C. The amount of steam to be used in the reboiler of the low-boiling separation column A was 3200 kg/hour.

For the operating conditions of the purification column B, the reflux ratio was 66.3, the reflux volume at the column top was 6300 kg/hour, and the temperature at the column bottom was 124°C. The amount of steam to be used in the reboiler of the purification column B was 2400 kg/hour.

Further, in the steady state of the continuous operation of the ethylene carbonate purification of Example 2, when the body column bottom liquid retention (L) of the low-boiling separation column A is defined as VA, the body column bottom liquid retention (L) of the purification column B is defined as VB, the amount of the side cut component (Bs) to be withdrawn (t/hour) is defined as BsV, the column body inner diameter (cm) of the low-boiling separation column A is defined as DA, the column kettle inner diameter (cm) of the low-boiling separation column A is defined as Da, the column body inner diameter (cm) of the purification column B is defined as DB, and the column kettle inner diameter (cm) of the purification column B is defined as Db, VA/BsV was 73, VB/BsV was 73, Da/BsV was 19, Db/BsV was 19, DA/Da was 4.4, and DB/Db was 4.

In Example 2, the low-boiling separation column A (first column) was specialized in removing the low-boiling component by the method of continuously introducing crude ethylene carbonate into the low-boiling separation column A and continuously withdrawing the low-boiling component (column top component (At)) and the column bottom component (Ab) containing an ethylene carbonate for distillation and separation without withdrawal through the side cut, so that the column height could be shortened, and the capital investment could be reduced. Further, the low-boiling separation column A was specialized in removing the low-boiling component, so that there was room in the column load, and the introduced liquid could be sufficiently separated as crude ethylene carbonate with specifications equivalent to an industrial grade with a purity of ethylene carbonate of 99.5 mass%. Then, slight amounts of the low-boiling and high-boiling components remaining could be removed by continuously introducing the column bottom component (Ab) into the purification column B having the side outlet and withdrawing ethylene carbonate through the side outlet, so that ethylene carbonate of electronic grade could be produced. Then, since most of the low-boiling component had been removed in the low-boiling separation column A (first column), there was room for the column load in the purification column B (second column), and the energy consumption for separation could be decreased. Further, since the pressure loss in the column can be reduced due to no increase in column load, and the temperature at the column bottom can be lowered, the amounts of the low-boiling and high-boiling by-products generated by decomposition, polymerization reaction, and the like can be reduced in the purification column B, as a result of which the amount of waste could be reduced.

Thus, an ethylene carbonate with electronic grade specifications could be produced efficiently on an industrial scale in Example 2.

[Example 3]

In the same manner as in Example 1, ethylene carbonate was purified using the low-boiling separation column A and the purification column B according to the process flow shown in Figure 1. However, the packings of the low-boiling separation column A and the purification column B were each Mellapak.

The introduced liquid continuously introduced into the low-boiling separation column A was a crude ethylene carbonate with specifications equivalent to an industrial grade with a purity of ethylene carbonate of 99.5 mass%. In Example 3, the material of the low-boiling separation column A was stainless steel, and the theoretical number of stages was 25 stages. In the low-boiling separation column A, the crude ethylene carbonate was introduced from the 14th stage position from the top of the theoretical stages. The material of the purification column B was stainless steel, the theoretical number of stages was 9 stages, and it had a side outlet. In the purification column B, the column bottom component (Ab) of the low-boiling separation column A was introduced from the 7th stage position from the top of the theoretical stages. In the purification column B, the side outlet was located at the 3rd stage position from the top of the theoretical stages.

The crude ethylene carbonate was introduced into the low-boiling separation column A at a flow rate of 5350 kg/hour, and the flow rate of the low-boiling component (column top component (At)) to be withdrawn through the column top of the low-boiling separation column A was 510 kg/hour. The column bottom component (Ab) containing an ethylene

carbonate with a purity of 99.72 mass% was withdrawn through the column bottom of the low-boiling separation column A at a ratio of 4840 kg/hour and introduced into the purification column B. In the purification column B, the flow rate of the low-boiling component (column top component (Bt)) to be withdrawn through the column top was 95 kg/hour, and the flow rate of the high-boiling component (column bottom component (Bb)) to be withdrawn through the column bottom was 50 kg/hour. In the purification column B, ethylene carbonate was withdrawn at a ratio of 4695 kg/hour (BsV = 4.695 t/hour) through the side outlet as the side cut component (Bs). In the purification column B, the ethylene carbonate withdrawn through the side outlet had a purity of 99.991 mass%, a DEG concentration of 25 mass ppm, a content of each of ethylene oxide and ethylene glycol of 30 mass ppm or less, a water content of 20 mass ppm or less, and a content of each of the nine metal components of sodium, potassium, copper, iron, lead, zinc, chromium, nickel, and calcium of 1 $\mu$g/mL or less, thereby satisfying the specifications (electronic grade specifications) for electrolyte solution applications.

For the operating conditions of the low-boiling separation column A, the reflux ratio was 14.5, the reflux volume at the column top was 7400 kg/hour, and the temperature at the column bottom was 128°C. The amount of steam to be used in the reboiler of the low-boiling separation column A was 3200 kg/hour.

For the operating conditions of the purification column B, the reflux ratio was 66.3, the reflux volume at the column top was 6300 kg/hour, and the temperature at the column bottom was 126°C. The amount of steam to be used in the reboiler of the purification column B was 2400 kg/hour.

Further, in the steady state of the continuous operation of the ethylene carbonate purification of Example 3, when the body column bottom liquid retention (L) of the low-boiling separation column A is defined as VA, the body column bottom liquid retention (L) of the purification column B is defined as VB, the amount of the side cut component (Bs) to be withdrawn (t/hour) is defined as BsV, the column body inner diameter (cm) of the low-boiling separation column A is defined as DA, the column kettle inner diameter (cm) of the low-boiling separation column A is defined as Da, the column body inner diameter (cm) of the purification column B is defined as DB, and the column kettle inner diameter (cm) of the purification column B is defined as Db, VA/BsV was 53, VB/BsV was 53, Da/BsV was 19, Db/BsV was 19, DA/Da was 4.4, and DB/Db was 4.

In Example 3, the low-boiling separation column A (first column) was specialized in removing the low-boiling component by the method of continuously introducing crude ethylene carbonate into the low-boiling separation column A and continuously withdrawing the low-boiling component (column top component (At)) and the column bottom component (Ab) containing an ethylene carbonate for distillation and separation without withdrawal through the side cut, so that the column height could be shortened, and the capital investment could be reduced. Further, the low-boiling separation column A was specialized in removing the low-boiling component, so that there was room in the column load, and the introduced liquid could be sufficiently separated as crude ethylene carbonate with specifications equivalent to an industrial grade with a purity of ethylene carbonate of 99.5 mass%. Then, slight amounts of the low-boiling and high-boiling components remaining could be removed by continuously introducing the column bottom component (Ab) into the purification column B having the side outlet and withdrawing ethylene carbonate through the side outlet, so that ethylene carbonate of electronic grade could be produced. Then, since most of the low-boiling component had been removed in the low-boiling separation column A (first column), there was room for the column load in the purification column B (second column), and the energy consumption for separation could be decreased. Further, since the pressure loss in the column could be reduced due to no increase in column load, and the temperature at the column bottom could be lowered, the amounts of the low-boiling and high-boiling by-products generated by decomposition, polymerization reaction, and the like could be reduced in the purification column B, as a result of which the amount of waste could be reduced.

Thus, an ethylene carbonate with electronic grade specifications could be produced efficiently on an industrial scale in Example 3.

[Example 4]

In the same manner as in Example 1, ethylene carbonate was purified using the low-boiling separation column A and the purification column B according to the process flow shown in Figure 1.

In the same manner as in Example 1, the introduced liquid continuously introduced into the low-boiling separation column A was a crude ethylene carbonate with specifications equivalent to an industrial grade with a purity of ethylene carbonate of 99.5 mass%. In Example 4, the material of the low-boiling separation column A was stainless steel, and the theoretical number of stages was 25 stages. In the low-boiling separation column A, the crude ethylene carbonate was introduced from the 14th stage position from the top of the theoretical stages. The material of the purification column B was stainless steel, the theoretical number of stages was 9 stages, and it had a side outlet. In the purification column B, the column bottom component (Ab) of the low-boiling separation column A was introduced from the 7th stage position from the top of the theoretical stages. In the purification column B, the side outlet was located at the 3rd stage position from the top of the theoretical stages.

The crude ethylene carbonate was introduced into the low-boiling separation column A at a flow rate of 5350 kg/hour, and the flow rate of the low-boiling component (column top component (At)) to be withdrawn through the column top of the low-boiling separation column A was 500 kg/hour. The column bottom component (Ab) containing an ethylene carbonate with a purity of 99.73 mass% was withdrawn at a ratio of 4850 kg/hour through the column bottom of the low-boiling separation column A and introduced into the purification column B. In the purification column B, the flow rate of the low-boiling component (column top component (Bt)) to be withdrawn through the column top was 90 kg/hour, and the flow rate of the high-boiling component (column bottom component (Bb)) to be withdrawn through the column bottom was 50 kg/hour. In the purification column B, ethylene carbonate was withdrawn at a ratio of 4710 kg/hour (BsV = 4.710 t/hour) through the side outlet as the side cut component (Bs). In the purification column B, the ethylene carbonate withdrawn through the side outlet had a purity of 99.994 mass%, a DEG concentration of 15 mass ppm, a content of each of ethylene oxide and ethylene glycol of 30 mass ppm or less, a water content of 20 mass ppm or less, and a content of each of the nine metal components of sodium, potassium, copper, iron, lead, zinc, chromium, nickel, and calcium of 1 μg/mL or less, thereby satisfying the specifications (electronic grade specifications) for electrolyte solution applications.

For the operating conditions of the low-boiling separation column A, the reflux ratio was 14.8, the reflux volume at the column top was 7400 kg/hour, and the temperature at the column bottom was 124°C. The amount of steam to be used in the reboiler of the low-boiling separation column A was 3200 kg/hour.

For the operating conditions of the purification column B, the reflux ratio was 70.0, the reflux volume at the column top was 6300 kg/hour, and the temperature at the column bottom was 124°C. The amount of steam to be used in the reboiler of the purification column B was 2400 kg/hour.

Further, in the steady state of the continuous operation of the ethylene carbonate purification of Example 4, when the body column bottom liquid retention (L) of the low-boiling separation column A is defined as VA, the body column bottom liquid retention (L) of the purification column B is defined as VB, the amount of the side cut component (Bs) to be withdrawn (t/hour) is defined as BsV, the column body inner diameter (cm) of the low-boiling separation column A is defined as DA, the column kettle inner diameter (cm) of the low-boiling separation column A is defined as Da, the column body inner diameter (cm) of the purification column B is defined as DB, and the column kettle inner diameter (cm) of the purification column B is defined as Db, VA/BsV was 73, VB/BsV was 73, Da/BsV was 19, Db/BsV was 19, DA/Da was 4.4, and DB/Db was 4.

In Example 4, the low-boiling separation column A (first column) was specialized in removing the low-boiling component by the method of continuously introducing crude ethylene carbonate into the low-boiling separation column A and continuously withdrawing the low-boiling component (column top component (At)) and the column bottom component (Ab) containing an ethylene carbonate for distillation and separation without withdrawal through the side cut, so that the column height could be shortened, and the capital investment could be reduced. Further, the low-boiling separation column A was specialized in removing the low-boiling component, so that there was room in the column load, and the introduced liquid could be sufficiently separated as crude ethylene carbonate with specifications equivalent to an industrial grade with a purity of ethylene carbonate of 99.5 mass%. Then, slight amounts of the low-boiling and high-boiling components remaining could be removed by continuously introducing the column bottom component (Ab) into the purification column B having the side outlet and withdrawing ethylene carbonate through the side outlet, so that ethylene carbonate of electronic grade could be produced. Then, since most of the low-boiling component had been removed in the low-boiling separation column A (first column), there was room for the column load in the purification column B (second column), and the energy consumption for separation could be decreased. Further, since the pressure loss in the column could be reduced due to no increase in column load, and the temperature at the column bottom could be lowered, the amounts of the low-boiling and high-boiling byproducts generated by decomposition, polymerization reaction, and the like could be reduced in the purification column B, as a result of which the amount of waste could be reduced.

Thus, an ethylene carbonate with electronic grade specifications could be produced efficiently on an industrial scale in Example 4.

[Comparative Example 1]

Figure 2 is a schematic view of the process flow of the production method for ethylene carbonate used in Comparative Example 1. In Figure 2, A is a low-boiling separation column that continuously distills and separates ethylene carbonate from the low-boiling component, and B is a purification column that continuously distills and separates the ethylene carbonate into three components of a column top component (Bt), a side cut component (Bs), and a column bottom component (Bb). A2 is a column top condenser of the low-boiling separation column A, and A5 is a reboiler of the low-boiling separation column A. Likewise, B2 is a column top condenser of the purification column B, and B5 is a reboiler of the purification column B. The reboiler type of the low-boiling separation column A was of forced circulation, and the reboiler type of the purification column B was of forced circulation. Further, a regular packing was packed in

each of the low-boiling separation column A and the purification column B.

The introduced liquid continuously introduced into the low-boiling separation column A was a crude ethylene carbonate with specifications equivalent to an industrial grade with a purity of ethylene carbonate of 99.5 mass%. In Comparative Example 1, the material of the low-boiling separation column A was stainless steel, and the theoretical number of stages was 25 stages, and it had a side outlet. In the low-boiling separation column A, the crude ethylene carbonate was introduced from the 14th stage position from the top of the theoretical stages. In the low-boiling separation column A, the side outlet was located at the 13th stage position from the top of the theoretical stages. The material of the purification column B was stainless steel, and the theoretical number of stages was 9 stages, and it had a side outlet. In the purification column B, the column bottom component (Ab) of the low-boiling separation column A was introduced from the 7th stage position from the top of the theoretical stages. In the purification column B, the side outlet was located at the 2nd stage position from the top of the theoretical stages.

The crude ethylene carbonate was introduced into the low-boiling separation column A at a flow rate of 1500 kg/hour, and the flow rate of the low-boiling component (column top component (At)) to be withdrawn through the column top of the low-boiling separation column A was 135 kg/hour. The amount of the column bottom component withdrawn through the column bottom of the low-boiling separation column A was 10 kg/hour. Through the side outlet of the low-boiling separation column A, a component containing an ethylene carbonate with a purity of 99.93 mass% was withdrawn at a ratio of 1355 kg/hour and introduced into the purification column B. In the purification column B, the flow rate of the low-boiling component (column top component (Bt)) to be withdrawn through the column top was 25 kg/hour, and the flow rate of the high-boiling component (column bottom component (Bb)) to be withdrawn through the column bottom was 5 kg/hour. In the purification column B, ethylene carbonate was withdrawn at a ratio of 1325 kg/hour (BsV = 1.325 t/hour) through the side outlet as the side cut component (Bs). In the purification column B, the ethylene carbonate withdrawn through the side outlet had a purity of 99.99 mass% and a DEG concentration of 55 mass ppm. Therefore, an electronic grade specification of a DEG concentration of 50 mass ppm or less was not satisfied, which was out of specifications for electrolyte solution applications.

For the operating conditions of the low-boiling separation column A, the reflux ratio was 14.8, the reflux volume at the column top was 2000 kg/hour, and the temperature at the column bottom was 124°C. The amount of steam to be used in the reboiler of the low-boiling separation column A was 900 kg/hour.

For the operating conditions of the purification column B, the reflux ratio was 70.0, the reflux volume at the column top was 1750 kg/hour, and the temperature at the column bottom was 124°C. The amount of steam to be used in the reboiler of the purification column B was 670 kg/hour.

Further, in the steady state of the continuous operation of the ethylene carbonate purification of Comparative Example 1, when the body column bottom liquid retention (L) of the low-boiling separation column A is defined as VA, the body column bottom liquid retention (L) of the purification column B is defined as VB, the amount of the side cut component (Bs) to be withdrawn (t/hour) is defined as BsV, the column body inner diameter (cm) of the low-boiling separation column A is defined as DA, the column kettle inner diameter (cm) of the low-boiling separation column A is defined as Da, the column body inner diameter (cm) of the purification column B is defined as DB, and the column kettle inner diameter (cm) of the purification column B is defined as Db, VA/BsV was 73, VB/BsV was 73, Da/BsV was 19, Db/BsV was 19, DA/Da was 4.4, and DB/Db was 4.

[Comparative Example 2]

In the same manner as in Comparative Example 1, ethylene carbonate was purified using the low-boiling separation column A and the purification column B according to the process flow shown in Figure 2.

The introduced liquid continuously introduced into the low-boiling separation column A was a crude ethylene carbonate with specifications equivalent to an industrial grade with a purity of ethylene carbonate of 99.5 mass%. In Comparative Example 2, the material of the low-boiling separation column A was stainless steel, the theoretical number of stages was 35 stages, and it had a side outlet. In the low-boiling separation column A, the crude ethylene carbonate was introduced from the 31st stage position from the top of the theoretical stages. In the low-boiling separation column A, the side outlet was located at the 23rd stage position from the top of the theoretical stages. The material of the purification column B was stainless steel, and the theoretical number of stages was 9 stages, and it had a side outlet. In the purification column B, the column bottom component (Ab) of the low-boiling separation column A was introduced from the 7th stage position from the top of the theoretical stages. In the purification column B, the side outlet was located at the 2nd stage position from the top of the theoretical stages.

The crude ethylene carbonate was introduced into the low-boiling separation column A at a flow rate of 1500 kg/hour, and the flow rate of the low-boiling component (column top component (At)) to be withdrawn through the column top of the low-boiling separation column A was 140 kg/hour. The flow rate of the high-boiling component to be withdrawn through the column bottom of the low-boiling separation column A was 15 kg/hour. A component containing an ethylene carbonate with a purity of 99.92 mass% was withdrawn at a ratio of 1345 kg/hour through the side outlet of the low-boiling separation column A and introduced into the purification column B. In the purification column B, the flow rate of the low-boiling component (column top component (Bt)) to be withdrawn through the column top was 25 kg/hour, and the flow rate of the high-boiling component (column bottom component (Bb)) to be withdrawn through the column bottom was 5 kg/hour. In the purification column B, ethylene carbonate was withdrawn at a ratio of 1315 kg/hour (BsV = 1.315 t/hour) through the side outlet as the side cut component (Bs). In the purification column B, the ethylene carbonate withdrawn through the side outlet had a purity of 99.99 mass% and a DEG concentration of 15 mass ppm.

For the operating conditions of the low-boiling separation column A, the reflux ratio was 15.0, the reflux volume at the column top was 2100 kg/hour, and the temperature at the column bottom was 128°C. The amount of steam to be used in the reboiler of the low-boiling separation column A was 920 kg/hour. Thus, in Comparative Example 2, the theoretical number of stages in the low-boiling separation column A was increased to 35 stages in order to obtain an ethylene carbonate with electronic grade specifications, so that the pressure loss in the column was large, and the temperature at the column bottom was as high as 128°C, as a result of which the amount of steam to be used in the reboiler was as large as 920 kg/hour, as compared with Comparative Example 1, resulting in a large energy cost. Further, despite such a large energy cost, the amount of the side cut component (Bs) to be withdrawn (BsV: production volume of ethylene carbonate) was as small as "1315 kg/hour", as compared with "1325 kg/hour" in Example 1.

For the operating conditions of the purification column B, the reflux ratio was 70.0, the reflux volume at the column top was 1750 kg/hour, and the temperature at the column bottom was 124°C. The amount of steam to be used in the reboiler of the purification column B was 670 kg/hour.

Further, in the steady state of the continuous operation of the ethylene carbonate purification of Comparative Example 2, when the body column bottom liquid retention (L) of the low-boiling separation column A is defined as VA, the body column bottom liquid retention (L) of the purification column B is defined as VB, the amount of the side cut component (Bs) to be withdrawn (t/hour) is defined as BsV, the column body inner diameter (cm) of the low-boiling separation column A is defined as DA, the column kettle inner diameter (cm) of the low-boiling separation column A is defined as Da, the column body inner diameter (cm) of the purification column B is defined as DB, and the column kettle inner diameter (cm) of the purification column B is defined as Db, VA/BsV was 73, VB/BsV was 73, Da/BsV was 19, Db/BsV was 19, DA/Da was 4.4, and DB/Db was 4.

[Comparative Example 3]

In the same manner as in Comparative Examples 1 and 2, ethylene carbonate was purified using the low-boiling separation column A and the purification column B according to the process flow shown in Figure 2. However, a batch purification column for ethylene carbonate was used in Figure 2 as the purification column B in Comparative Example 3. Others were the same as Comparative Examples 1 and 2.

The introduced liquid continuously introduced into the low-boiling separation column A was a crude ethylene carbonate with specifications equivalent to an industrial grade with a purity of ethylene carbonate of 99.5 mass%. In Comparative Example 3, the material of the low-boiling separation column A was stainless steel, the theoretical number of stages was 25 stages, and it had a side outlet. In the low-boiling separation column A, the crude ethylene carbonate was introduced from the 14th stage position from the top of the theoretical stages. In the low-boiling separation column A, the side outlet was located at the 13th stage position from the top of the theoretical stages. The material of the purification column B was stainless steel, and the theoretical number of stages was 9 stages, and it had a side outlet. In the purification column B, the column bottom component (Ab) of the low-boiling separation column A was introduced from the 7th stage position from the top of the theoretical stages. In the purification column B, the side outlet was located at the 2nd stage position from the top of the theoretical stages.

The crude ethylene carbonate was introduced into the low-boiling separation column A at a flow rate of 1500 kg/hour, and the flow rate of the low-boiling component (column top component (At)) to be withdrawn through the column top of the low-boiling separation column A was 135 kg/hour. The amount of the column bottom component withdrawn through the column bottom of the low-boiling separation column A was 10 kg/hour. A component containing an ethylene carbonate with a purity of 99.93 mass% was withdrawn through the side outlet of the low-boiling separation column A at a ratio of 1355 kg/hour and introduced into the purification column B.

For the operating conditions of the low-boiling separation column A, the reflux ratio was 14.9, the reflux volume at the column top was 2015 kg/hour, and the temperature at the column bottom was 124°C. Into the purification column B, 18 tons of the liquid withdrawn through the side outlet of the low-boiling separation column A was fed, and after total reflux for 1.5 hours at a reflux volume of 6000 kg/hour, the low-boiling component was withdrawn at a flow rate of 1300 kg/hour to be withdrawn for 2 hours. Thereafter, 13.2 tons of ethylene carbonate with a purity of 99.992 mass% (with electronic grade specifications) was recovered. Thus, it turned out that the recovery efficiency of an ethylene carbonate with electronic grade specifications was poor in Comparative Example 3. The high-boiling component remaining in the column kettle of the purification column B was 2.0 tons. For the operating conditions of the purification column B, the reflux ratio was 4.6, and the temperature at the column bottom was 124 to 130°C.

This application is based on Japanese Patent Application No. 2021-065963, filed on April 8, 2021, the contents of which are incorporated herein by reference.

Industrial Applicability

The method for industrially producing a cyclic alkylene carbonate of the present invention enables cyclic alkylene carbonate with electronic grade specifications to be produced efficiently. Further, the cyclic alkylene carbonate obtained by the production method of the present invention is useful, for example, as a lithium battery electrolyte solution.

Reference Signs List

A: Low-boiling separation column
B: Cyclic alkylene carbonate purification column
A2: Column top condenser of low-boiling separation column A
A5: Reboiler of low-boiling separation column A
B2: Column top condenser of cyclic alkylene carbonate purification column B
B5: Reboiler of cyclic alkylene carbonate purification column B

**Claims**

1. A method for industrially producing a cyclic alkylene carbonate, comprising:

   a first distillation and separation step of continuously introducing a crude cyclic alkylene carbonate into a low-boiling separation column A, and continuously withdrawing a column top component (At) through a column top and a column bottom component (Ab) containing a cyclic alkylene carbonate through a column bottom in the low-boiling separation column A; and
   a second distillation and separation step of continuously introducing the column bottom component (Ab) obtained in the first distillation and separation step into a cyclic alkylene carbonate purification column B having a side outlet (side cut outlet), and continuously withdrawing three components of a column top component (Bt) through a column top, a side cut component (Bs) through the side outlet, and a column bottom component (Bb) through a column bottom in the purification column B,
   wherein the side cut component (Bs) is a cyclic alkylene carbonate with electronic grade specifications.

2. The production method according to claim 1, wherein the crude cyclic alkylene carbonate is a crude ethylene carbonate.

3. The production method according to claim 1 or 2, wherein the crude cyclic alkylene carbonate is a crude ethylene carbonate that is obtained using ethylene oxide and carbon dioxide as starting materials.

4. The production method according to any one of claims 1 to 3, wherein, when a body column bottom liquid retention (L) of the low-boiling separation column A is defined as VA, a body column bottom liquid retention (L) of the purification column B is defined as VB, and an amount of the side cut component (Bs) to be withdrawn (t/hour) is defined as BsV, the conditions of formulas (1) and (2) below are satisfied:

   $$10 \leq VA/BsV \leq 1000 \cdots (1);$$

   and

   $$10 \leq VB/BsV \leq 1000 \cdots (2).$$

5. The production method according to any one of claims 1 to 4, wherein, when a column kettle inner diameter (cm) of the low-boiling separation column A is defined as Da, a column kettle inner diameter (cm) of the purification column B is defined as Db, and the amount of the side cut component (Bs) to be withdrawn (t/hour) is defined as

BsV, the conditions of formulas (3) and (4) below are satisfied:

$$10 \leq Da/BsV \leq 50 \cdots (3);$$

and

$$10 \leq Db/BsV \leq 50 \cdots (4).$$

**6.** The production method according to any one of claims 1 to 5, wherein, when a column body inner diameter (cm) of the low-boiling separation column A is defined as DA, the column kettle inner diameter (cm) of the low-boiling separation column A is defined as Da, a column body inner diameter (cm) of the purification column B is defined as DB, the column kettle inner diameter (cm) of the purification column B is defined as Db, the conditions of formulas (5) and (6) below are satisfied:

$$2 \leq DA/Da \leq 10 \cdots (5);$$

and

$$2 \leq DB/Db \leq 10 \cdots (6).$$

**7.** The production method according to any one of claims 1 to 6, wherein a reboiler type in the low-boiling separation column A and/or the purification column B is of forced circulation, cross pipe falling film, or thin film evaporation.

**8.** The production method according to any one of claims 1 to 7, wherein

a regular packing is packed in the low-boiling separation column A and/or the purification column B, and
the regular packing is any of Mellapak, Gempak, Technopack, Flexipac, Sulzer packing, Goodroll packing, Glitch grid, and gauze packing.

[Figure 1]

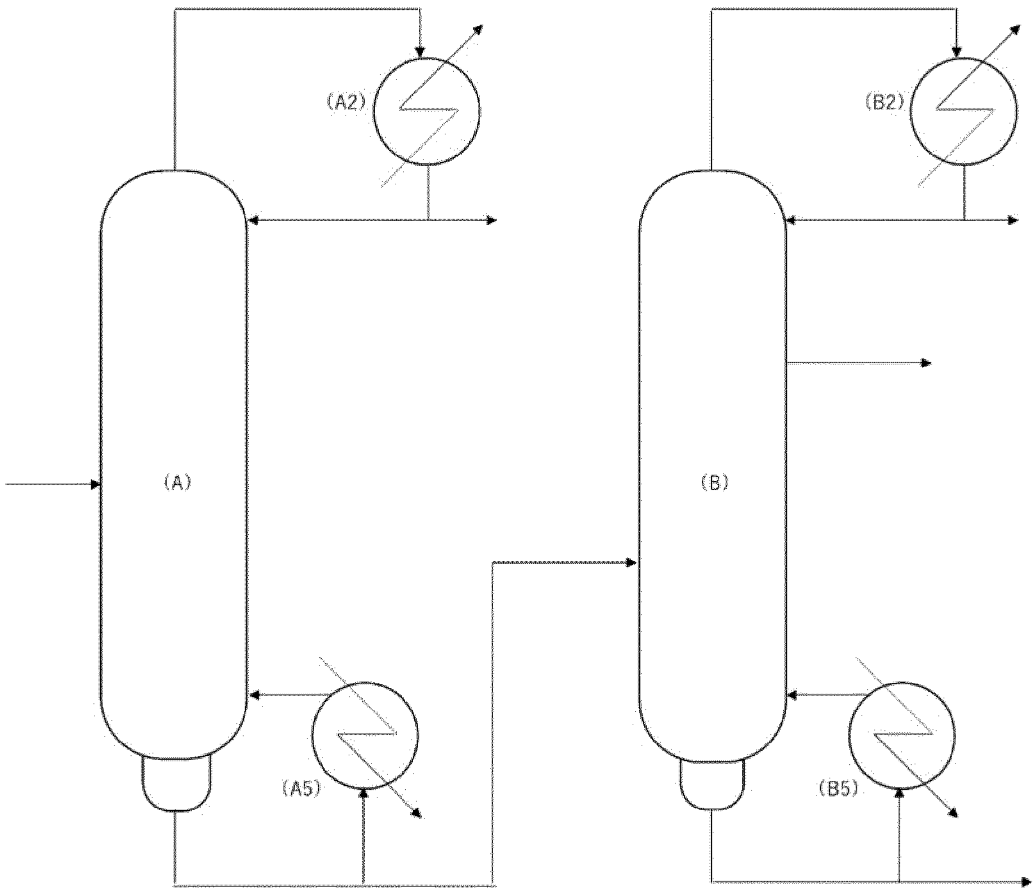

[Figure 2]

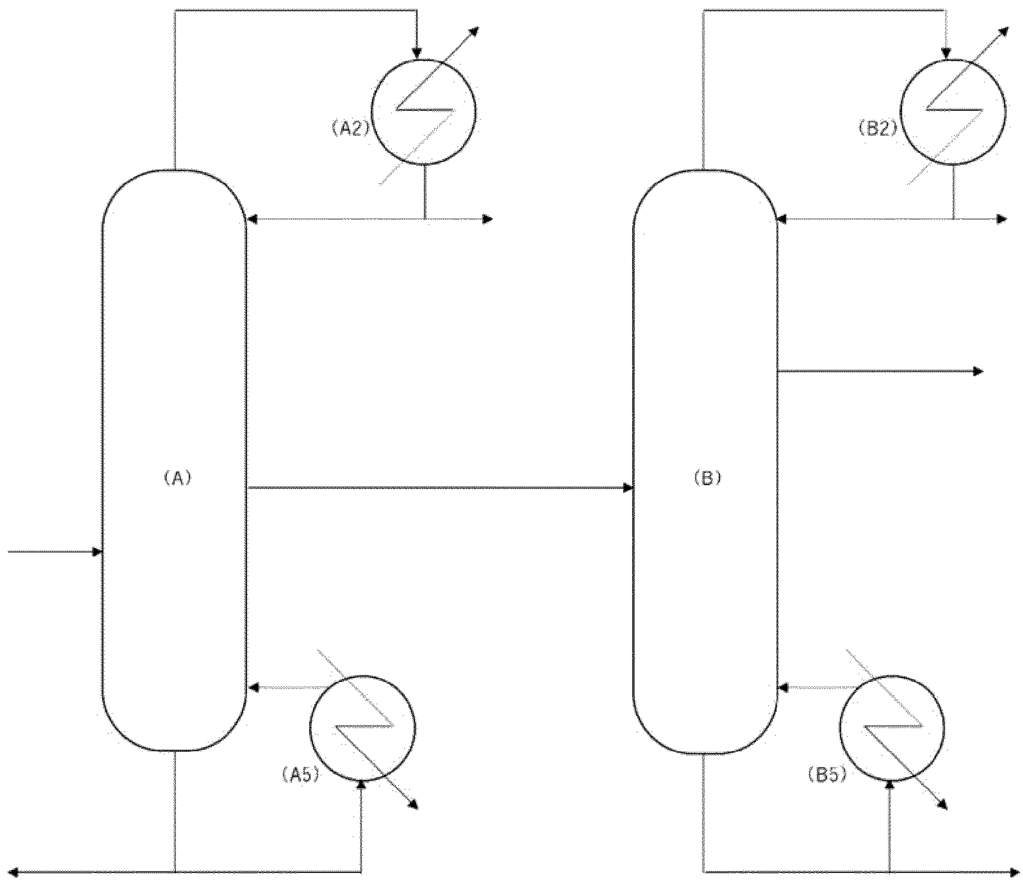

## INTERNATIONAL SEARCH REPORT

International application No. **PCT/JP2021/043988**

**A. CLASSIFICATION OF SUBJECT MATTER**

***C07D 317/38***(2006.01)i
FI: C07D317/38

According to International Patent Classification (IPC) or to both national classification and IPC

**B. FIELDS SEARCHED**

Minimum documentation searched (classification system followed by classification symbols)

C07D317/38

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2006-104096 A (ASAHI KASEI CHEMICALS CORP) 20 April 2006 (2006-04-20) claims, examples, fig. 1 | 1-8 |
| A | JP 2006-104095 A (ASAHI KASEI CHEMICALS CORP) 20 April 2006 (2006-04-20) claims, examples, fig. 1 | 1-8 |
| A | JP 2003-176283 A (MITSUBISHI CHEMICALS CORP) 24 June 2003 (2003-06-24) claims, examples, fig. 1, 2 | 1-8 |
| A | WO 2019/125151 A1 (ALTA INNOVATION SUPPORT B.V.) 27 June 2019 (2019-06-27) claims, fig. 1 | 1-8 |
| A | US 4166773 A (UNION CARBIDE CORPORATION) 04 September 1979 (1979-09-04) fig. 1, claims | 1-8 |
| A | KR 10-0809877 B1 (ECOPRO CO. LTD.) 27 February 2008 (2008-02-27) claims, fig. 1 | 1-8 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 January 2022** | **18 January 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2021/043988**

**C. DOCUMENTS CONSIDERED TO BE RELEVANT**

| Category* | Citation of document, with indication, where appropriate, of the relevant passages | Relevant to claim No. |
|---|---|---|
| A | US 2002/0147349 A1 (HUNTSMAN PETROCHEMICAL CORPORATION) 10 October 2002 (2002-10-10)<br>fig. 1, claims | 1-8 |

Form PCT/ISA/210 (second sheet) (January 2015)

**INTERNATIONAL SEARCH REPORT**
**Information on patent family members**

International application No.
**PCT/JP2021/043988**

| Patent document cited in search report | Publication date (day/month/year) | Patent family member(s) | Publication date (day/month/year) |
|---|---|---|---|
| JP 2006-104096 A | 20 April 2006 | (Family: none) | |
| JP 2006-104095 A | 20 April 2006 | (Family: none) | |
| JP 2003-176283 A | 24 June 2003 | WO 2003/006418 A1 | |
| WO 2019/125151 A1 | 27 June 2019 | JP 2021-508736 A<br>US 2020/0399239 A1<br>EP 3728213 A1<br>KR 10-2020-0091433 A<br>CN 111615510 A | |
| US 4166773 A | 04 September 1979 | JP 54-89974 A<br>GB 2010685 A<br>DE 2855258 A<br>FR 2412329 A | |
| KR 10-0809877 B1 | 27 February 2008 | (Family: none) | |
| US 2002/0147349 A1 | 10 October 2002 | WO 2000/020407 A1<br>EP 1119561 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 106588862
- CN 208260225
- WO 2004108696 A
- JP 2021065963 A